# EUROPÄISCHE PATENTSCHRIFT

(11) **EP 0 868 181 B1**
(45) Veröffentlichungstag und Bekanntmachung des Hinweises auf die Patenterteilung: **31.05.2000**
(21) Anmeldenummer: 96943125.3
(22) Anmeldetag: 14.12.1996
(51) Int. Cl.: A61K 31/445

(54) **VERWENDUNG DES ALPHA,ALPHA-DIPHENYLESSIGSÄURE-4-(N-METHYL-PIPERIDYL)ESTERS ALS SPASMOANALGETIKUM**
USE OF ALPHA,ALPHA-DIPHENYLACETIC ACID-4-(N-METHYL-PIPERIDYL)ESTER AS ANTI-SPASMODIC
UTILISATION D'ACIDE ALPHA,ALPHA-DIPHENYLACETIQUE -4-(N-METHYL-PIPERIDYL)ESTER COMME ANTISPASMODIQUE

(30) Priorität: 20.12.1995 DE 19547621
(43) Veröffentlichungstag der Anmeldung: 07.10.1998
(73) Patentinhaber: Pein, Eckhart, 37154 Northeim (DE); Ritter, Helmut, 42111 Wuppertal (DE); Laven, Reinhard, 38259 Salzgitter (DE)
(72) Erfinder: Pein, Eckhart, 37154 Northeim (DE); Ritter, Helmut, 42111 Wuppertal (DE); Laven, Reinhard, 38259 Salzgitter (DE)
(74) Vertreter: Hrabal, Ulrich, Dr.
(86) Internationale Anmeldenummer: EP9605627
(87) Internationale Veröffentlichungsnummer: WO9722343

(56) Entgegenhaltungen:
- EP-A- 0 335 758
- EP-A- 0 445 731
- DE-A- 4 205 843
- US-A- 4 678 780
- AM.J.PHYSIOL., Bd. 250, Nr. 1 Pt. 1, 1986, Seiten G50-G59, XP000645243 R.J.GILBERT ET AL.: "Effect of selective muscarinic antagonists on peristaltic contractions in opossum smooth muscle"
- J.MED.CHEM., Bd. 29, Nr. 8, 1986, Seiten 1512-1516, XP000647301 J.A.WATERS ET AL.: "Anticonvulsant Activity of Piperidinol and (Dialkylamino)alkanol Esters"
- J.MED.CHEM., Bd. 15, Nr. 5, 1972, Seiten 506-509, XP000647308 D.F.BIGGS ET AL.: "Stereochemical Studies of Antimuscarinic Agents. Diastereoisomeric Esters of 3-Tropanol, 1,3-Dimethyl-4-piperidinol, and Related Compounds"
- NAUNYN-SCHMIEDEBERG'S ARCH. PHARMACOL., Bd. 339, Nr. 1-2, 1989, Seiten 145-151, XP000645257 P.A.LUCCHESI ET AL.: "Interaction of agonists and selective antagonists with gastric smooth muscle muscarinic receptors" in der Anmeldung erwähnt

## Beschreibung

Der vorliegenden Erfindung liegt die Aufgabe zugrunde, eine Substanz aufzuzeigen, die zur Behandlung von schmerzhaften Spasmen der glatten Muskulatur im Bereich der Cavitas abdominalis, insbesondere in der Bauchfellhöhle, wie Gallenkoliken, Magen- und Darmspasmen, Reizkolon, Pylorusspasmus sowie im Retroperitonealraum wie Nierenkoliken und Uretersteinkoliken geeignet ist.

Die bisherigen Kenntnisse über die pharmakologische Wirkung von α,α-Diphenylessigsäure-4-(N-methyl-piperidyl)-ester, im folgenden auch als der "Ester" bezeichnet, bezog sich weitgehend auf seine cholinolytische Aktivität, die seit dem Jahre 1943 bekannt ist (J. Am. Chem. Soc., 65 (1943) 262-267). In der deutschen Patentschrift 42 05 843 C2 wurde beschrieben, daß der α,α-Diphenylessigsäure-4-(N-methyl-piperidyl)-ester eine starke neurotrop-spasmolytische, d.h. cholinolytische Wirkung am Harnblasenmuskel, der anatomisch und physiologisch zu den Beckeneingeweiden gehört, sowie eine geringe zentrale cholinolytische Aktivität entfaltet,

Bei der Überprüfung der weiteren pharmakodynamischen Eigenschaften des α,α-Diphenylessigsäure-4-(N-methyl-piperidyl)-esters ergaben sich überraschend und unerwartet eine ausgeprägte muskulotrop-spasmolytische Wirkung auf die Organe der Cavitas abdominalis (Bauchhöhle) sowie erhebliche analgetische Eigenschaften der Verbindung.

Die Erfindung betrifft daher die Verwendung von α,α-Diphenylessigsäure-4-(N-methyl-piperidyl)-ester oder dessen Salzen mit physiologisch verträglichen organischen oder anorganischen Säuren zur Herstellung von Arzneimitteln zur Therapie schmerzhafter muskulärer Krampfzustände, im Bereich der Cavitas abdominalis, insbesondere von muskulären Krampfzuständen im Bereich der Bauchfellhöhle wie Pylorusspasmus, Gallenkolik, Magen- und Darmspasmen, Reizkolon sowie im Retroperitonealraum wie Nierenkoliken und Uretersteinkoliken. Die Verwendung kann in der Human- und in der Veterinärmedizin erfolgen.

Beispiele für verwendbare physiologisch verträgliche Säuren zur Verwendung der Salze sind die üblicherweise in der Pharmakologie verwendeten organischen und anorganischen Säuren, wie Salzsäure, Schwefelsäure, Phosphorsäure oder Carbonsäure und Dicarbonsäuren. Beispiele für geeignete organische Säuren sind Essigsäure, Fettsäuren wie Stearinsäure, Laurinsäure, Ölsäure oder Palmitinsäure, Glykolsäure, Milchsäure, Brenztraubensäure, Malonsäure, Bernsteinsäure, Fumarsäure, Apfelsäure, Weinsäure, Zitronensäure, Cyclamensäure, Ascorbinsäure, Benzoesäure, 4-Hydroxybenzoesäure, Zimtsäure, Salizylsäure, Mandelsäure; es sind auch Sulfonsäuren geeignet wie Methansulfonsäure, Ethansulfonsäure und Hydroxyethansulfonsäure. Die Salze können auf übliche Weise gebildet werden.

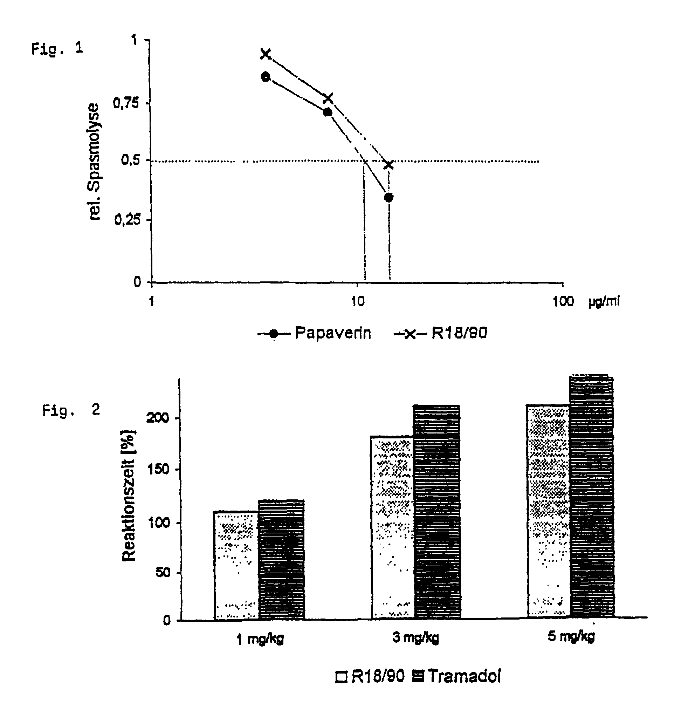

In Fig. 1 ist die muskulotrop-spasmolytische Wirksamkeit des Esters an der isolierten Taenia coli des Meerschweinchens im Vergleich zu Papaverin dargestellt. Als Spasmodikum diente Bariumchlorid in einer Badkonzentration von 0,2 mg/ml. Dargestellt ist die Dosis-Wirkungs-Beziehung zur relativen Spasmolyse.

Der EC₅₀-Vert für den Ester lag bei 12,6 µg/ml und erreichte die Wirkungsstärke des Papaverins.

Unerwartet und überraschend war insbesondere, daß α,α-Diphenylessigsäure-4-(N-methyl-piperidyl)-ester auch eine starke analgetische Wirkkomponente besitzt. In Fig. 3 wird die analgetische Wirkung des Esters im Vergleich zur Referenzsubstanz Tramadol wiedergegeben. Die Bestimmung der analgetischen Aktivität des Esters erfolgt mit Hilfe der üblichen hot-plate-Technik. Der Ester zeigte bei der subcutanen Applikation an Mäusen (20-25 g Körpergewicht) eine dosisabhängige analgetische Wirkung, die in ihrer Wirkstärke der des Tramadols entsprach.

Die überraschenden und unerwarteten pharmakodynamischen Effekte des α,α-Diphenylessigsäure-4-(N-methyl-piperidyl)-esters zeichnen diese Substanz als ein Spasmoanalgetikum aus, das eine wirksame Behandlung von Krankheitszuständen im Bereich der Cavitas abdominalis ermöglicht, die durch schmerzhafte, muskuläre Krampfzustände gekennzeichnet sind (Gallenkoliken, Magen- und Darmspasmen, Reizkolon, Pylorusspasmus, Nieren- und Ureterkoliken, schmerzhafte Tenesmen durch Bestrahlung im Abdominal- und Retroperitonealbereich).

In Fig. 2 ist der analgetische Effekt des Esters im Vergleich zu Tramadol im hot-plate-Test nach subcutaner Applikation an der Maus dargestellt.

Die Vorteilhaftigkeit des Esters liegt in dem Umstand, daß die aufgezeigte Substanz die Wirkqualität eines Analgetikums mit der eines muskulotrop wirkenden Spasmolytikums in sich vereint.

Der erfindungsgemäß verwendete Ester kann auf verschiedene Weise verabreicht werden, beispielsweise oral oder parenteral. Sie kann beispielsweise in Form von Pulvern, Tabletten, Kapseln, Dragees, Zäpfchen oder in wäßriger bzw. öliger Suspension verabreicht werden. Im folgenden sind einige Formulierungsbeispiele angegeben.

Der erfindungsgemäß verwendete Ester kann zusammen mit weiteren therapeutisch wirksamen Substanzen eingesetzt werden, wie Antibiotika und Beruhigungsmittel (Tranquillantien).

### Beispiele

### 1. Tabletten zu 200 mg

| | |
|---|---|
| α,α-Diphenylessigsäure-4-(N-methyl-piperidyl)-ester | 20 mg |
| mikrokristalline Zellulose | 159 mg |
| quervernetztes Poly-(N-vinylpyrrolidon) | 20 mg |
| Magnesiumstearat | 1 mg |

### 2. Kapseln zu 300 mg

| | |
|---|---|
| α,α-Diphenylessigsäure-4-(N-methyl-piperidyl)-ester | 20 mg |
| gelbes Bienenwachs | 10 mg |
| Sojabohnenöl | 10 mg |
| Pflanzenöl | 160 mg |
| Kapselhülle | 100 mg |

### 3. Injektionsampullen

| | |
|---|---|
| α,α-Diphenylessigsäure-4-(N-methyl-piperidyl)-ester | 20 mg |
| physiol. NaCl-Lsg. ad | 2 ml |

## Patentansprüche

1. Verwendung von α,α-Diphenylessigsäure-4-(N-methyl-piperidyl)-ester zur Herstellung von Arzneimitteln zur Behandlung schmerzhafter muskulärer Krampfzustände im Bereich der Cavitas abdominalis.

2. Verwendung nach Anspruch 1 zur Herstellung von Arzneimitteln zur Behandlung von Pylorusspasmus, Gallenkolik, Nierenkolik, Uretersteinkolik, Magen- und Darmspasmen, Reizkolon.

3. Verwendung nach Anspruch 1 oder 2 zusammen mit mindestens einer weiteren therapeutisch wirksamen Substanz.

4. Verwendung nach Anspruch 3, worin die weitere therapeutisch wirksame Substanz ein Antibiotikum oder ein Tranquillantium ist.

## Claims

1. The use of α,α-diphenylacetic acid-4-(N-methyl-piperidyl) ester for the production of drugs for treating painful muscular cramp conditions in the region of the abdominal cavity.

2. A use according to claim 1 for the production of drugs for treating pylorospasm, biliary colic, renal colic, urethral stone colic, stomach and intestinal spasms, irritable bowel syndrome.

3. A use according to claims 1 or 2 together with at least one further therapeutically effective substance.

4. A use according to claim 3, wherein the further therapeutically effective substance is an antibiotic or a tranquilliser.

## Revendications

1. Utilisation de l'ester 4-(N-méthylpipéridylique) de l'acide α,α-diphénylacétique pour préparer des médicaments destinés au traitement des états de crampes musculaires douloureux dans le domaine de la cavité abdominale.

2. Utilisation selon la revendication 1, pour préparer des médicaments destinés au traitement du spasme du pylore, des coliques biliaires, des coliques néphrétiques, des coliques dues à un calcul urétéral, des spasmes de l'estomac et de l'intestin, du côlon irritable.

3. Utilisation selon la revendication 1 ou 2, couplée à au moins une autre substance à effet thérapeutique.

4. Utilisation selon la revendication 3, dans laquelle l'autre substance à effet thérapeutique est un antibiotique ou un tranquillisant.
